# EUROPEAN PATENT SPECIFICATION

(11) **EP 2 388 011 B1**
(45) Date of publication and mention of the grant of the patent: **04.04.2018**
(21) Application number: 11166917.2
(22) Date of filing: 20.05.2011
(51) Int. Cl.: A61K 36/28, A61K 36/899, A61P 39/06

(54) **ANTIOXIDANT COMPOSITION FOR REDUCING OXIDATIVE STRESS IN DOGS**
ANTIOXIDANS-ZUSAMMENSETZUNG ZUR REDUZIERUNG VON OXIDATIVEM STRESS IN HUNDE
COMPOSITION ANTIOXIDANTE POUR LA RÉDUCTION DU STRESS OXYDATIF CHEZ LES CHIENS

(30) Priority: 21.05.2010 IT MI20100926
(43) Date of publication of application: 23.11.2011
(73) Proprietor: DRN S.r.l., 26020 Palazzo Pignano (IT)
(72) Inventor: Conselvan, Patrizio, 350 31 ABANO TERME (IT); Falanga, Gennaro, 26024 GERRE DE' CAPRIOLI (IT)
(74) Representative: Villa, Livia

(56) References cited:
- WO-A1-02/062329
- US-A1- 2005 181 047
- US-A1- 2006 233 774
- US-A1- 2007 116 779
- Protocol for Life: "The Professional Educator", Protocol for Life Balance , April 2009 (2009-04), XP002617811, Retrieved from the Internet: URL:http://www.protocolforlife.com/idc/gro ups/public/documents/general_information/0 74552.pdf [retrieved on 2011-01-19]
- DUARTE-ALMEIDA J M ET AL: "Phenolic composition and antioxidant activity of culms and sugarcane (Saccharum officinarum L.) products", FOOD CHEMISTRY, ELSEVIER LTD, NL, vol. 125, no. 2, 19 September 2010 (2010-09-19), pages 660-664, XP027454072, ISSN: 0308-8146, DOI: 10.1016/J.FOODCHEM.2010.09.059 [retrieved on 2010-09-19]
- JOAQUIM MAURÍCIO DUARTE-ALMEIDA ET AL: "Antioxidant Activity of Phenolics Compounds From Sugar Cane (Saccharum officinarum L.) Juice", PLANT FOODS FOR HUMAN NUTRITION, KLUWER ACADEMIC PUBLISHERS, DO, vol. 61, no. 4, 22 November 2006 (2006-11-22), pages 187-192, XP019453674, ISSN: 1573-9104, DOI: 10.1007/S11130-006-0032-6
- VILA, FABIANA C.; COLOMBO, RENATA; LIRA, TATIANA O. DE AND YARIWAKE, JANETE H..: "HPLC Microfractionation of Flavones and Antioxidant (Radical Scavenging) Activity of Saccharum officinarum L.", JOURNAL OF THE BRAZILIAN CHEMICAL SOCIETY, vol. 19, no. 5, 2008, pages 903-908, XP000002656419, São Paulo ISSN: 0103-5053, DOI: 10.1590/S0103-50532008000500013

## Description

### FIELD OF THE INVENTION

The present invention concerns an antioxidant composition for reducing oxidative stress in dogs. In particular, this composition has proved to be particularly suitable in that, as well as being surprisingly effective, it is extremely well tolerated by the body.

### STATE OF THE ART

The concept of "oxidative stress" is known to refer to the over-production and subsequent accumulation of free radicals in the body.

The free radical is a strongly oxidizing, highly unstable and very reactive species with typically a very short average lifespan, consisting of a molecule (formed by one or more atoms) having one unpaired electron. The latter is responsible for the high reactivity of the free radical which then tends to react to form other radicals, by removing an electron or a hydrogen atom from nearby molecules. The ensuing direct effect is to propagate the production of unstable and highly reactive molecules.

Over-production of these oxidizing species, in the absence of an adequate defence system, can trigger a veritable cascade of reactions which are highly damaging to the cell and compromise its structural and functional integrity.

An attack on the cell membrane by free radicals causes an alteration in cell membrane permeability. Damage can also be done to DNA, triggering alterations to the genome that can cause the onset of chromosomal mutations and degenerative processes in the cell. As has often been emphasized, under certain conditions free radicals can cause injury to cells and tissues, leading to the onset of chronic degenerative, cardiovascular and chronic inflammatory alterations which are responsible for various pathological conditions.

Oxidative damage depends on the co-existence of various factors: the intrinsic characteristics of the chemical structure of free radicals, the structural and functional properties of the molecular substratum with which they come into contact, and finally the organism's own defensive antioxidant capability. It is therefore clear that any condition that can cause an alteration in the physiological balance between production and removal of free radicals and a deficiency in the body's own antioxidant defence mechanisms, leads to the onset of a state of oxidative stress and the consequent appearance of pathological events.

Under physiological conditions and hence a state of adequate general wellbeing of the body, there is a precise balance between production and removal of free radicals, associated with an adequate defensive capability of the body, known as the antioxidant barrier.

The antioxidant barrier plays a predominant role in the defensive action displayed by the body against free radical attack. In fulfilling its antioxidant activity, it uses various molecules and enzyme systems of both exogenous origin, i.e. introduced into the body by means of either adequate nutrition or as synthetic preparations, and endogenous origin, i.e. produced directly by the cell's physiological metabolism.

These molecules with antioxidant action are able to neutralize free radicals as soon as they are formed, or at a later time, i.e. only after their oxidative action has already initiated.

Natural enzyme systems with antioxidant activity are represented by superoxide-dismutase (SOD), a metalloprotein, whose function is to remove the superoxide radical anion (O₂), by glutathione peroxidase (GPx) which has a reducing action on organic hydroperoxides utilizing glutathione as co-substrate, and finally by catalase (CAT) which reduces hydrogen peroxide (H₂O₂).

Also involved in this defensive activity are vitamins, polyphenols, trace elements and various other substances which are consumed with an adequate and proper diet.

However, pathological states such as chronic inflammatory, degenerative or neoplastic diseases, very intense physical activity, intake of pharmaceutical drugs, toxins, infections and/or advanced age, can give rise to oxidative stress, namely an imbalance between the production of reactive chemical species (free radicals) and the physiological defensive capacity (antioxidants). In other words, these conditions can induce a significant production of free radicals, to the extent that the physiological mechanisms are unable to run them or can undermine the efficiency of the physiological antioxidant systems.

Oxidative stress is itself a disease state, independent of the reason which has caused it and capable of significantly interfering with the treatment administered to an individual or with his state of health.

Since the assessment of oxidative stress in the canine species is of recent interest to veterinary medicine, the object of the present invention is therefore to find an effective remedy which is well tolerated by the animal's body, is able to reduce oxidative stress and presents the lowest possible toxicity as well as a high bioavailability.

### SUMMARY OF THE INVENTION

This object is achieved by the antioxidant composition as claimed in claim 1, comprising a polyphenol extract of sugar cane and silymarin, wherein in said extract at least 50% of polyphenols have a number average molecular weight of 600 to 1000 Daltons.

In a further aspect, the present invention concerns the use of said composition for treating oxidative stress in dogs.

As will be evident from the following detailed description, the composition of the invention has surprisingly shown an exceptionally high antioxidant power, such as to enable oxidative stress to be reduced, for example in dogs, by the suitable combination of its components which are well tolerated by the organism as well as being totally natural in origin.

The characteristics and advantages of the present invention will be evident from the following detailed description, and from the working examples provided for illustrative purposes.

### DETAILED DESCRIPTION OF THE INVENTION

The present invention therefore relates to an antioxidant composition comprising a polyphenol extract of sugar cane and silymarin, wherein in said extract at least 50% of polyphenols have a number average molecular weight of 600 to 1000 Daltons. It has actually been noted that the combination of these components surprisingly and advantageously enables the formation and propagation of free radicals to be successfully counteracted, hence proving to be particularly effective in the treatment of oxidative stress, especially oxidative stress in dogs, as will be even more apparent from the examples below.

Said sugar cane pertains to the genus *Saccharum* and can be one of the following species: *Saccharum arundinaceum, Saccharum bengalense, Saccharum edule, Saccharum munja, Saccharum officinarum, Saccharum procerum, Saccharum ravennae, Saccharum robustum, Saccharum sinense, Saccharum spontaneum,* preferably *Saccharum officinarum.*

Preferably, in said polyphenol extract, at least 70% of polyphenols have a number average molecular weight of 600 to 1000 Daltons, more preferably, at least 70% of polyphenols have a number average molecular weight of 650 to 800 Daltons.

In the polyphenol extract of sugar cane, the polyphenols present are natural antioxidants which act to prevent oxidation of lipoproteins and to sequester free radicals. However, a recognised problem associated with consumption of polyphenols deriving from plant foods in general, is their low bioavailability. The inventors of the present invention have surprisingly found that by combining said polyphenols with silymarin not only unexpected and significant results are achieved in terms of antioxidant activity, but also high bioavailability and tolerability.

Silymarin is a natural mixture mainly comprising three flavonolignans, namely silybin (also known as silybinin), silychristin and silydianin. This complex of flavonolignans is present in various plants, above all in milk thistle, wherein said flavonolignans are present in a ratio of about 3:1:1 respectively (silybin is hence considered to be the most important component under both quantitative and qualitative terms). Silymarin is not present in leaves, but is concentrated in the seeds and the protein layer on the external surface of the fruit.

Therefore, in a preferred embodiment, in the antioxidant composition of the present invention, said silymarin is in the form of a milk thistle extract.

Preferably, the antioxidant composition of the present invention comprises 20 to 60% by weight of polyphenol extract of sugar cane and 0.5 to 5% by weight of silymarin, as it is noted that further improved results are obtained in terms of oxidative stress reduction.

More preferably, the antioxidant composition of the present invention comprises 25 to 55% by weight of polyphenol extract of sugar cane and 1 to 3% by weight of silymarin, as it is noted that excellent results are obtained in terms of oxidative stress reduction already shortly after consumption of the composition.

Even more preferably, the antioxidant composition of the present invention comprises 35 to 55% by weight of polyphenol extract of sugar cane and 1 to 2.5% by weight of silymarin, as it enables the best result to be obtained for all the observed parameters.

According to another preferred embodiment, said composition further comprises vitamin C, vitamin E, vitamin A or mixtures thereof. In fact, it has been noted that the additional presence of these compounds promotes oxidative stress reduction. Preferably, said composition further comprises vitamin C, vitamin E or mixtures thereof.

More preferably, said composition further comprises 0.5 to 10% by weight of vitamin C and 5 to 15% by weight of vitamin E, and even more preferably, 1 to 6% by weight of vitamin C and 6 to 14% by weight of vitamin E.

Optionally, the composition of the invention further comprises pharmaceutically or veterinarily acceptable excipients.

According to a particularly preferred embodiment of the invention, the antioxidant composition consists of a polyphenol extract of sugar cane, silymarin, vitamin C, vitamin E and pharmaceutically or veterinarily acceptable excipients.

Suitable pharmaceutically or veterinarily acceptable excipients are maltodextrin, sugars, starches, cellulose and its derivatives, dried yeasts, magnesium carbonate, magnesium stearate, colloidal silica or mixtures thereof.

In another aspect, the present invention relates to a process for preparing the above described antioxidant composition, comprising the step of mixing the above described components.

It should be understood that all the aspects identified as preferred and advantageous for the antioxidant composition, are accordingly considered to be preferred and advantageous for the preparation process of the present invention. In accordance with a preferred embodiment, said process further comprises a step of compressing the mixture of the antioxidant composition to form a tablet.

In another aspect, the present invention concerns an antioxidant composition for use as a medicament. In particular, said antioxidant composition is preferably used in the veterinary field.

In accordance with a preferred embodiment, said antioxidant composition is used for reducing oxidative stress in dogs. In this respect, as will be seen from the working examples to follow, the composition of the invention has shown a surprising antioxidant effect, derivable from the selected combination of the its components.

Preferably, said antioxidant composition is to be administered orally. More preferably, said antioxidant composition is in tablet form. Preferably, the antioxidant composition of the invention can be in unit dose form comprising 300-1200 mg of polyphenol extract of sugar cane and 7.5-75 mg of silymarin. More preferably, said unit dose comprises 525-825 mg of polyphenol extract of sugar cane and 15-37.5 mg of silymarin.

In accordance with another preferred embodiment, said unit dose further comprises vitamin C, vitamin E or mixtures thereof. More preferably, said unit dose further comprises 7.5-150 mg of vitamin C and 75-225 mg of vitamin E; and even more preferably, 15-90 mg of vitamin C and 90-210 mg of vitamin E.

Particularly preferred embodiments are unit doses which comprise:
- 800 mg of polyphenol extract of sugar cane, 100 mg of silymarin, 40 mg of vitamin C, 20 mg of vitamin E;
- 400 mg of polyphenol extract of sugar cane, 180 mg of silymarin, 60 mg of vitamin C, 35 mg of vitamin E;
- 600 mg of polyphenol extract of sugar cane, 200 mg of silymarin, 80 mg of vitamin C, 35 mg of vitamin E;
- 700 mg of polyphenol extract of sugar cane, 100 mg of silymarin, 20 mg of vitamin C, 30 mg of vitamin E;
- 550 mg of polyphenol extract of sugar cane, 50 mg of silymarin.

Working examples of the present invention are given hereinafter by way of nonlimiting illustration.

To this end, the oxidative stress of dogs in the examples was determined by using the-ROMs test and BAP test on blood material.

In particular, the d-ROMs (Reactive Oxygen Metabolites, ROM) test evaluates the total oxidizing capacity of a sample of plasma or serum, by using N,N-diethyl-para-phenylene diamine as the reducing reagent. Practically, a small aliquot of biological sample to be analyzed is dissolved in a buffer solution at acid pH; addition of the reagent is accompanied by a colour change of the solution, initially colourless then becoming pink. The resulting change in absorbance is measured photometrically, either in kinetic or endpoint mode. The test results are expressed in conventional units, i.e. CARR Units (CARR U), where 1 CARR corresponds to 0.08 mg/dl of a hydrogen peroxide solution.

The BAP (Biological Antioxidant Potential) test instead enables the efficiency of the antioxidant barrier in plasma to be determined in terms of iron-reducing activity, from ferric ions to ferrous ions. The evaluation is determined photometrically by means of a chromogen with decolourization caused by the shift from ferric ions (Fe³⁺) to ferrous ions (Fe²⁺). The plasma sample to be analysed is dissolved in a coloured solution obtained by adding a source of ferric ions (FeCl₃, ferric chloride) to a chromogen (sulphur-based compound). After a five-minute incubation period the solution decolourizes; the intensity of this process is proportional to the ability of the tested plasma to reduce ferric irons. The antioxidant power of the photometrically tested plasma is indicated in microM and is inversely proportional to the increase in measured units.

### EXAMPLES

### Example 1

### Preparation of the compositions of the invention

The following antioxidant compositions were prepared according to the present invention, all in unit doses of 1,500 mg:

| | Sugar cane polyphenols | Vitamin E | Vitamin C | Dry extract of milk thistle containing 85% silymarin | Excipients |
|---|---|---|---|---|---|
| Composition A | 800 mg | 100 mg | 40 mg | 20 mg | 540 mg |
| Composition B | 400 mg | 180 mg | 60 mg | 35 mg | 825 mg |
| Composition C | 600 mg | 200 mg | 80 mg | 35 mg | 585 mg |
| Composition D | 700 mg | 100 mg | 20 mg | 30 mg | 650 mg |
| Composition E | 550 mg | | | 50 mg | 900 mg |

These compositions were obtained by mixing the components in a rotary mixer at high speed until a homogeneous mixture was obtained. This mixture was then conveyed to a rotary tablet press where the compositions in tablet form were obtained.

### Example 2.

### Determination of the antioxidant power of the compositions of the invention in dogs well on in years (12 to 18 years)

25 elderly dogs (12 to 18 years) were selected, some with chronic diseases in progress, others in good health but with clear signs of both physical and mental ageing. The clinical parameters considered were:
- appetite,
- hair shine,
- cognitive state, and
- mobility.

Each of these parameters was assigned a score from 1 to 4:
1: poor
2: sufficient
3: good
4: excellent.

The main data for each subject are listed in table 1.

These were subdivided into three groups according to their weight:
- from no. 1 to no. 10 (20 - 30 kg)
- from no. 11 to no. 20 (10 - 20 kg)
- from no. 21 to no. 25 (1-10 kg)

**Table 1. Characteristics of the 25 selected dogs**

| | Weight (kg) | Age | Disease (if present) | Appetite | Hair shine | Cognitive state | Mobility |
|---|---|---|---|---|---|---|---|
| 1 | 20 | 17 | Mammary tumour | 3 | 2 | 3 | 1 - 2 |
| 2 | 21 | 18 | | 2 | 2 | 1 | 2 |
| 3 | 28 | 14 | | 2 | 2 | 2 | 1 |
| 4 | 22 | 15 | Mammary tumour | 1 | 1 | 2 | 2 |
| 5 | 20 | 12 | | 3 | 2 | 2 | 1 |
| 6 | 23 | 12 | | 2 | 2 | 1 | 2 |
| 7 | 22 | 13 | | 3 | 1 | 1 | 2 |
| 8 | 24 | 12 | Osteoarthritis of hip | 2 | 2 | 1 | 1 |
| 9 | 20 | 13 | Mammary tumour | 1 | 1 | 2 | 1 |
| 10 | 23 | 12 | | 3 | 1 | 1 | 1 |
| 11 | 12 | 14 | Leishmaniasis | 2 | 1 | 2 | 1 |
| 12 | 11 | 15 | Leishmaniasis | 2 | 2 | 2 | 2 |
| 13 | 14 | 15 | | 1 | 1 | 1 | 1 |
| 14 | 11 | 12 | | 2 | 2 | 3 | 1 |
| 15 | 15 | 13 | | 1 | 1 | 2 | 2 |
| 16 | 12 | 12 | | 3 | 3 | 1 | 1 |
| 17 | 12 | 15 | Lymphoma in remission | 2 | 1 | 3 | 3 |
| 18 | 15 | 12 | | 1 | 1 | 1 | 1 |
| 19 | 16 | 16 | | 3 | 1 | 1 | 3 |
| 20 | 16 | 12 | | 2 | 2 | 1 | 2 |
| 21 | 3 | 17 | Leishmaniasis | 2 | 2 | 2 | 2 |
| 22 | 6 | 16 | | 2 | 1 | 1 | 2 |
| 23 | 4 | 13 | Cardiopathy (cl. ISAAC 3) | 1 | 1 | 2 | 2 |
| 24 | 4 | 15 | | 2 | 2 | 1 | 2 |
| 25 | 5 | 13 | | 2 | 1 | 1 | 2 |

All the dogs were then treated with the unit dose of composition A of the invention, as from Example 1. The scheduled dosage was one unit dose per day for 60 days.

The following clinical and laboratory parameters were then assessed in the dogs:
- Pro-oxidant parameters:
   Reactive oxygen metabolites (free radical derivatives) - dROM test; Reactive oxygen metabolites (ROM) are considerably more stable than radicals and can hence be measured. High values indicate oxidative stress.
- Anti-oxidant parameters:
   1. Plasma thiol groups - SHp test
      Thiols are a significant component of the plasma antioxidant barrier (natural). Indeed, the -SH groups of these molecules (often of protein type) react chemically with radicals to inactivate them.
      Low values for this analysis therefore signify that the natural antioxidant activity is poor and that oxidative stress is present.
   2. Plasma barrier to oxidation induced by hypochlorite - Oxy adsorbent test
      The test measures the general antioxidant capacity of serum by measuring its capacity to oppose the powerful oxidative action of hypochlorous acid, with which it is processed.
      Low values for this analysis signify a reduction in antioxidant power of the natural barrier and hence an increase in oxidative stress. These values are to be regarded as directly correlated to the degree of damage and hence the severity of stress.
   3. Biological antioxidant potential - BAP Test
      As with the previous, the purpose of this test is to measure the capacity of the natural antioxidant barrier, but with a different technique. The significance of the values is therefore perfectly comparable.

The results are summarized in Table 2 (laboratory tests before and after 60 days of treatment with the composition of the invention) and in Table 3 (clinical parameters after 60 days of treatment).

*Reference ranges* (i.e. values within which the subjects do not present symptoms of oxidative stress):
- d-ROM: 250-300 CARR Units (understood to be the oxidizing capacity of a range from 20,000 to 24,000 mg of H₂O₂)
- SHp: 450 to 650 µmol/l
- OXY test: > 350 µmol HCIO/I
- BAP test: > 2200 µmol/l

**Table 2: Results before and after 60 days of treatment with composition A of the invention**

| | Disease (if present) | d-ROM I (T₀) | d-ROM II (T₂) | SHp I (T₀) | SHp II (T₂) | OXY I (T₀) | OXY II (T₂) | BAP I (T₀) | BAP II (T₂) |
|---|---|---|---|---|---|---|---|---|---|
| 1 | Mammary tumour | 206 | 182 | *39* | 451 | *290* | 456 | *270* | *440* |
| 2 | | 245 | 135 | *81* | *187* | 466 | 467 | *1090* | *1168* |
| 3 | | *340* | 197 | *44* | *455* | *300* | 495 | *579* | *900* |
| 4 | Mammary tumour | *290* | 122 | *290* | 569 | *234* | 528 | *447* | 2402 |
| 5 | | *368* | 204 | *197* | 676 | *106* | 488 | *200* | *234* |
| 6 | | *280* | 189 | *236* | *250* | *136* | 380 | *335* | *780* |
| 7 | | *337* | 156 | *121* | *330* | *210* | 360 | *256* | *553* |
| 8 | Osteoarthritis of hip | *865* | *454* | *326* | 700 | *120* | 427 | *1280* | 2500 |
| 9 | Mammary tumour | *432* | 220 | *37* | 495 | *136* | 440 | *885* | 2290 |
| 10 | | *337* | 187 | *100* | 660 | *218* | 512 | *798* | *1555* |
| 11 | Leishmaniasis | *976* | *546* | *87* | *270* | *153* | 361 | *650* | *2100* |
| 12 | Leishmaniasis | *543* | *265* | *99* | *432* | *85* | *255* | *735* | *1755* |
| 13 | | *321* | 123 | *331* | 768 | *321* | 653 | *1545* | 2480 |
| 14 | | 221 | 176 | *415* | 854 | 285 | 398 | *1669* | 2890 |
| 15 | | *663* | *290* | *43* | *547* | *98* | *320* | *507* | *1800* |
| 16 | | *387* | 218 | *295* | 810 | *334* | 450 | *1675* | 2530 |
| 17 | Lymphoma in remission | *410* | 165 | *220* | 660 | *277* | 490 | *1200* | 2650 |
| 18 | | *385* | 249 | *300* | 759 | *280* | 432 | *1440* | 2376 |
| 19 | | 198 | 166 | *441* | 692 | *340* | 540 | *1256* | 2685 |
| 20 | | *520* | 190 | *280* | 730 | *227* | 465 | *1450* | 2210 |
| 21 | Leishmaniasis | 176 | 121 | 455 | 670 | *300* | 490 | *1760* | 2700 |
| 22 | | *429* | 196 | *375* | 745 | *275* | 409 | *1000* | 2290 |
| 23 | Cardiopathy (cl ISAAC 3) | *365* | 128 | *320* | 688 | *215* | 479 | *1742* | 2495 |
| 24 | | 232 | 200 | *436* | 821 | *304* | 428 | *1484* | 2280 |
| 25 | | *430* | 235 | *150* | *687* | *196* | 505 | 834 | 2400 |

Those values which fell outside the reference ranges, indicating oxidative stress, are shown in italics. In the first column, the values at Time 0 (T₀) and after 60 days (T₂) are given for each analyte in each of the tests.

**Table 3. Clinical assessment after 60 days of treatment with composition A of the invention**

| | Weight (kg) | Age | Disease (if present) | Appetite | Hair shine | Cognitive state | Mobility |
|---|---|---|---|---|---|---|---|
| 1 | 20 | 17 | Mammary tumour | 3 | 3 | 3 | 3 |
| 2 | 21 | 18 | | 3 | 3 | 3 | 4 |
| 3 | 28 | 14 | | 3 | 4 | 3 | 3 |
| 4 | 22 | 15 | Mammary tumour | 4 | 3 | 4 | 3 |
| 5 | 20 | 12 | | 3 | 3 | 4 | 3 |
| 6 | 23 | 12 | | 4 | 3 | 3 | 4 |
| 7 | 22 | 13 | | 3 | 2 | 2 | 3 |
| 8 | 24 | 12 | Osteoarthritis of hip | 3 | 3 | 4 | 3 |
| 9 | 20 | 13 | Mammary tumour | 3 | 3 | 4 | 3 |
| 10 | 23 | 12 | | 3 | 3 | 3 | 3 |
| 11 | 12 | 14 | Leishmaniasis | 2 | 2 | 3 | 3 |
| 12 | 11 | 15 | Leishmaniasis | 2 | 3 | 3 | 2 |
| 13 | 14 | 15 | | 3 | 2 | 3 | 2 |
| 14 | 11 | 12 | | 2 | 4 | 4 | 4 |
| 15 | 15 | 13 | | 2 | 2 | 3 | 2 |
| 16 | 12 | 12 | | 3 | 4 | 3 | 4 |
| 17 | 12 | 15 | Lymphoma in remission | 4 | 2 | 3 | 3 |
| 18 | 15 | 12 | | 3 | 2 | 3 | 3 |
| 19 | 16 | 16 | | 3 | 3 | 4 | 3 |
| 20 | 16 | 12 | | 4 | 4 | 3 | 3 |
| 21 | 3 | 17 | Leishmaniasis | 3 | 3 | 3 | 3 |
| 22 | 6 | 16 | | 2 | 3 | 3 | 4 |
| 23 | 4 | 13 | Cardiopathy (cl ISAAC 3) | 2 | 3 | 3 | 3 |
| 24 | 4 | 15 | | 2 | 3 | 3 | 4 |
| 25 | 5 | 13 | | 4 | 2 | 3 | 4 |

The clinical parameters under consideration were advantageously significantly improved in all the tested subjects, including in those where the serum levels of the measured substances did not fall within, but were close to, the reference range values. It was hence observed that not only highly significant results were obtained in only 60 days, but there were also concretely reasonable grounds to suppose that by extending the treatment period all the considered biochemical parameters would have normalized.

The role of oxidative stress in pathogenesis or in the response to therapy is known from the literature [1]. Those subjects affected by neoplastic diseases (i.e. 1, 2, 9, 17) all showed a marked improvement both from the clinical perspective and from laboratory results. On the other hand, oxidative stress is known to be very much involved both in mechanisms of carcinogenesis [2] and in the mortality of cancer patients.

It has also been shown [3] that the concentration of ROM is higher the greater the proliferative character of cancer and that individuals with the best oxidative balance (ROM to antioxidant ratio) respond better to chemotherapy [4].

Referring in particular to subject 8 with osteoarthritis of the hip, this dog showed clear clinical and serum level improvements; evidence exists in the literature on the direct correlation between oxidative stress and cartilage degeneration in osteoarthritic processes [5].

Significant results were also achieved for subject 23 with valvular cardiopathy of medium severity (cl. Isaach III). This dog showed considerable clinical improvements, and, after a long period in therapy, he returned to an undoubtedly better quality of life with no change to his pharmaceutical drug therapy. It is well known in the literature that oxidative stress can promote the activation of cellular apoptosis in cardiac muscle leading to cardiac load disorder, and therefore plays an important role in the progression of such diseases [6].

The subjects affected by Leishmaniasis (11, 12, 21) showed greater clinical than analytical improvements. There are not many published studies on said disease, but it has been established that the oxidative balance of subjects affected by this and other infectious diseases is greatly altered [7, 8].

The other subjects examined were all clinically healthy elderly dogs, but in poor general condition and/or affected by varying degrees of cognitive dysfunction due to elderly age (as in Table 1). It has been shown in man that oxidative damage is particularly severe in neurodegenerative diseases of the elderly people [9]. Even in elderly dogs the brain accumulates proteins and oxidized lipids, which may be responsible for neuronal dysfunction, together with an accumulation in their cytoplasm of free radicals due to both an increase in free radical production and a reduction in antioxidant systems [10, 11].

All the subjects examined in this first clinical study gained a substantial and significant improvement in their cognitive ability, showing greater responsiveness to stimuli, capacity and disposition for play, memory and learning ability.

Also advantageously improved, often even by three points, were those subjects (identified in Table 3 with score 1) who showed particularly severe symptoms (nocturnal vocalization, compulsive behaviour, and inappropriate urination and defecation).

Therefore, it followed that the use of the antioxidant composition of the invention could be a valuable complement in the treatment of many chronic diseases and even a possible supportive treatment of cognitive disorders in elderly subjects.

### Example 3

### Determination of the antioxidant potential of the compositions of the invention in sledge dogs

The dogs examined were sledge dogs engaged in long-distance activity with prolonged effort for several hours per day, fed on a very high energy diet, reared and trained in a Lappish environment.

Included in the study were sledge dogs aged between 1 and 8 years, taking part in sports competitions, with normal blood chemistry values. All the dogs lived in the same kennels and were fed the same high energy food. The dogs began training in September and the sporting season ended in early April.

The training period was divided into two periods.

The first period began in September and ended in late October, during which time the dogs were trained on the ground by pulling and driving a ballasted quad bike, over varying route lengths starting at 5 km and reaching 20 km by the end of October when temperatures approximated 0°C.

The second training period began in November to coincide with the first snowfall and ended in late December. In this second phase, the dogs were trained on snow covered routes at temperatures ranging from -10°C to -20°C. The environmental conditions were similar to those of the races, the end of the second period coinciding with the height of the sporting season.

In order to evaluate the oxidative state of the dogs at the different times in the season, three time periods were indicated:
- end of August - before the start of training: T₀ (the dogs had been resting for 3 months)
- end of October - between the two training periods (ground and snow): T₁
- end of December - end of the training period on snow and start of the racing period: T₂

The dogs were divided into two groups; i.e. a first group (Group 1) treated with the composition of the invention (Composition A of Example 1) and a second group (Group 2) was the control, both were homogeneous with respect to age, sex, genetic background. The dogs in the two groups were subjected to the same rearing, nutritional and training conditions.

Group 1 was formed of 6 subjects, whereas Group 2 was of 5 subjects. Composition A in tablet form was administered daily from early September to Group 1 orally.

### Results

Time 0 (T₀): all the dogs presented d-ROMs values between 70 and 130 CARR U (see Table 4)
Time 1 (T₁): it was shown that for the dogs of Group 1, i.e. treated with Composition A, there was a significant reduction in oxidative stress which was definitely greater than in the control Group 2.

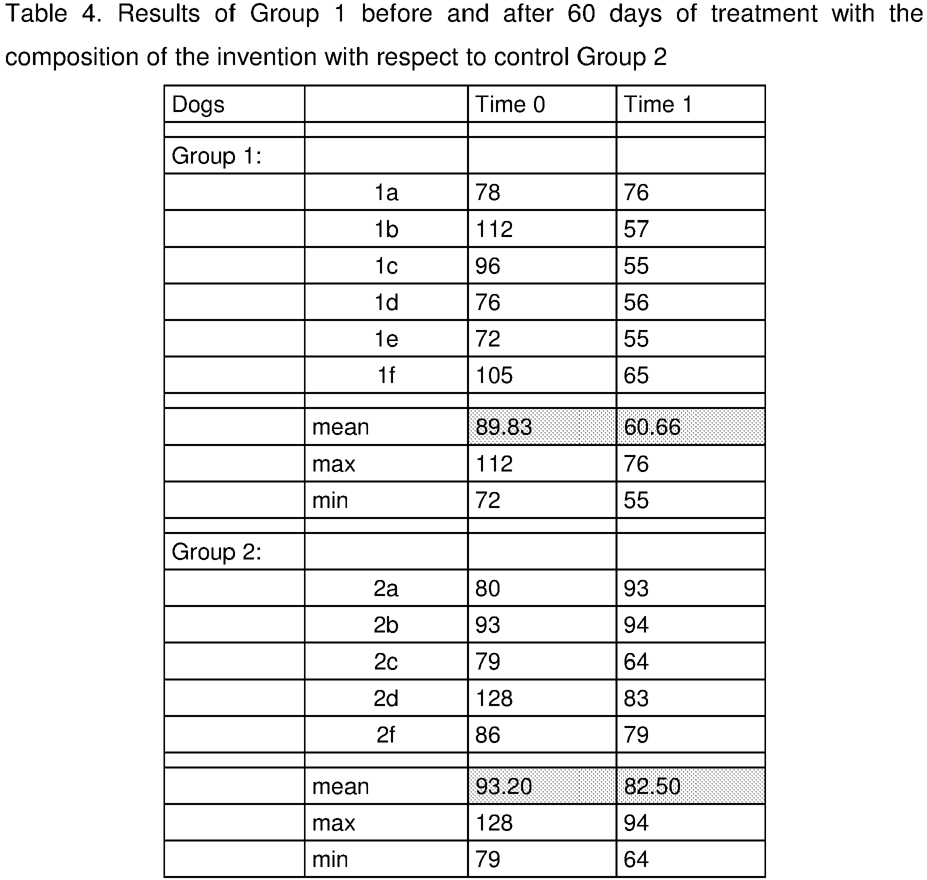

In light of the above results, it was confirmed that the oxidative stress values (d-ROMs) in sledge dogs treated with the antioxidant composition of the invention were reduced relative to those of sledge dogs left untreated. Indeed, it was observed that while in the latter group the mean reduction in oxidative stress was 11.48%, the mean reduction in oxidative stress in the Group 1 dogs was surprisingly 32.47%.

### Example 4

### Comparison between the compositions of the invention and known antioxidant compositions

Three comparative antioxidant compositions employed in the veterinary field were used, comprising as active ingredients:
- Comparative composition I: 50 mg vitamin E + 30 mg vitamin C
- Comparative composition II: 15 mg resveratrol + 30 mg Gingko biloba
- Comparative composition III: 30 mg blueberry + 45 mg Camellia sinensis These known compositions were compared with Composition E of the invention, as from Example 1:
- Composition E: 550 mg sugar cane polyphenols + 50 mg of dry extract of milk thistle containing 85% silymarin

The recovery times, ROS levels, and antioxidant activity levels were evaluated in adult dogs after aerobic physical activity, in hunting dogs after hunt activity, and in elderly dogs after moderate physical activity [12]. The selected subjects were all in good health with no diseases in progress. The various compositions with antioxidant activity were administered to all the dogs - 6 hunting dogs, 6 adult companion dogs and 6 elderly dogs - one week before the start of the test.

For each subject blood samples were collected on which the d-ROMs and BAP tests were carried out. The blood was collected one hour after physical activity, one day after and one week after.

**Table 5. Mean values for the three groups of dogs examined, results after 1 hour, 1 day and 1 week of treatment with the comparative compositions**

| | | Composition E | Comparative composition I | Comparative composition II | Comparative composition III |
|---|---|---|---|---|---|
| after 1 hour | | | | | |
| | d-ROM | 115 | 396 | 308 | 419 |
| | BAP | 1.529 | 1.315 | 1.153 | 997 |
| after 1 day | | | | | |
| | d-ROM | 84 | 285 | 196 | 341 |
| | BAP | 2.812 | 1.296 | 1.451 | 1.533 |
| after 1 week | | | | | |
| | d-ROM | 67 | 153 | 112 | 208 |
| | BAP | 4.253 | 2.003 | 2.546 | 1.614 |

In view of the fact that the following ranges are considered normal values:
- 56 - 92 CARR U. for d-ROMs test
- 1.224 - 3.279 µmoles/l for BAP test

it could be clearly seen that the invention Composition E surprisingly enabled notably great results to be achieved in terms of oxidative stress reduction, compared to known compositions for the same application, already 1 hour after administration. This indicates that high levels of effectiveness were found already shortly after consumption.

From the detailed description and the Examples above, the advantages of the composition of the present invention are clear. In particular, this composition has proved to be surprisingly and advantageously able to reduce oxidative stress in elderly dogs, in dogs suffering from various diseases as well as in dogs undergoing intense activity, while simultaneously having excellent tolerability by the organism and high bioavailability.

### Bibliographical references

1. Viviano KR et al. "Glutathione, cysteine, and ascorbate concentrations in clinically ill dogs and cats", J Vet Intern Med. 2009 Mar-Apr;23(2):250-7
2. Szczubiat M et al. "Oxidative stress parameters in bitches with mammary gland tumours", J Vet Med A Physiol Pathol Clin Med. 2004 Sep-Oct;51 (7-8):336-40
3. Vajdovich P et al. "Redox status of dogs with non-hodgkin lymphomas. An ESR study", Cancer Lett. 2005 Jun 28;224(2):339-46
4. Winter JL et al. "Antioxidant status and biomarkers of oxidative stress in dogs with lymphoma", J Vet Intern Med. 2009 Mar-Apr;23(2):311-6
5. Goranov NV "Serum markers of lipid peroxidation, antioxidant enzymatic defense, and collagen degradation in an experimental (Pond-Nuki) canine model of osteoarthritis", Vet Clin Pathol. 2007 Jun;36(2):192-5
6. Prasad K et al. "Oxidative stress as a mechanism of cardiac failure in chronic volume overload in canine model", J Mol Cell Cardiol. 1996 Feb;28(2):375-85
7. Bildik A et al. "Oxidative stress and non-enzymatic antioxidative status in dogs with visceral Leishmaniasis", Res Vet Sci. 2004 Aug;77(1):63-6
8. Kumar A et al. "A comparative study on oxidative stress in dogs infected with Ehrlichia canis with or without concurrent infection with Babesia gibsoni", Vet Res Commun. 2006 Nov;30(8):917-20
9. Head E et al. "Oxidative stress, aging, and central nervous system disease in the canine model of human brain aging", Vet Clin North Am Small Anim Pract. 2008 Jan;38(1):167-78, vi. Review
10. Hwang IK et al. "Differences in lipid peroxidation and Cu,Zn-superoxide dismutase in the hippocampal CA1 region between adult and aged dogs", J Vet Med Sci. 2008 Mar;70(3):273-7
11. Milgram NW et al. "Dietary enrichment counteracts age-associated cognitive dysfunction in canines", Neurobiol Aging. 2002 Sep-Oct;23(5):737-45
12. McMichael MA "Oxidative stress, antioxidants, and assessment of oxidative stress in dogs and cats", J Am Vet Med Assoc. 2007 Sep 1;231 (5):714-20. Review

## Claims

1. An antioxidant composition comprising a polyphenol extract of sugar cane and silymarin, wherein in said extract at least 50% of polyphenols have a number average molecular weight of 600 to 1000 Daltons.

2. The antioxidant composition of claim 1, wherein in said extract at least 70% of polyphenols have a number average molecular weight of 600 to 1000 Daltons.

3. The antioxidant composition of claim 2, wherein in said extract at least 70% of polyphenols have a number average molecular weight of 650 to 800 Daltons.

4. The antioxidant composition of any one of claims 1-3 comprising 20 to 60% by weight of polyphenol extract of sugar cane and 0.5 to 5% by weight of silymarin.

5. The antioxidant composition of claim 4 comprising 25 to 55% by weight of polyphenol extract of sugar cane and 1 to 3% by weight of silymarin.

6. The antioxidant composition of claim 5 comprising 35 to 55% by weight of polyphenol extract of sugar cane and 1 to 2.5% by weight of silymarin.

7. The antioxidant composition of any one of claims 1-6, further comprising vitamin C, vitamin E, vitamin A or mixtures thereof.

8. The antioxidant composition of claim 7 comprising 0.5 to 10% by weight of vitamin C and 5 to 15% by weight of vitamin E.

9. The antioxidant composition of claim 8 comprising 1 to 6% by weight of vitamin C and 6 to 14% by weight of vitamin E.

10. The antioxidant composition of any one of claims 1-9 for use in the treatment of oxidative stress in dogs.

11. The antioxidant composition of claim 10, to be administered orally, preferably in tablet form.

12. A unit dose of the antioxidant composition of any one of claims 1-7 comprising 300-1200 mg of polyphenol extract of sugar cane, 7.5-75 mg of silymarin, and optionally 7.5-150 mg of vitamin C and 75-225 mg of vitamin E.

13. The unit dose of claim 12, comprising 525-825 mg of polyphenol extract of sugar cane and 15-37.5 mg of silymarin.

14. The unit dose of claim 12 or 13, additionally comprising 7.5-150 mg of vitamin C and 75-225 mg of vitamin E.

15. The unit dose of claim 14, additionally comprising 15-90 mg of vitamin C and 90-210 mg of vitamin E.

## Patentansprüche

1. Antioxidative Zusammensetzung, umfassend einen Polyphenol- Extrakt aus Rohrzucker und Silymarin, wobei in dem Extrakt mindestens 50 % der Polyphenole ein durchschnittliches Molekulargewicht von 600 bis 1000 Dalton aufweisen.

2. Antioxidative Zusammensetzung nach Anspruch 1, wobei in dem Extrakt mindestens 70 % der Polyphenole ein durchschnittliches Molekulargewicht von 600 bis 1000 Dalton aufweisen.

3. Antioxidative Zusammensetzung nach Anspruch 2, wobei in dem Extrakt mindestens 70 % der Polyphenole ein durchschnittliches Molekulargewicht von 650 bis 800 Dalton aufweisen.

4. Antioxidative Zusammensetzung nach einem der Ansprüche 1-3 umfassend 20 bis 60 Gew.-% Polyphenol-Extrakt aus Rohrzucker und 0,5 bis 5 Gew.-% Silymarin.

5. Antioxidative Zusammensetzung nach Anspruch 4 umfassend 25 bis 55 Gew.-% Polyphenol-Extrakt aus Rohrzucker und 1 bis 3 Gew.-% Silymarin.

6. Antioxidative Zusammensetzung nach Anspruch 5 umfassend 35 bis 55 Gew.-% Polyphenol-Extrakt aus Rohrzucker und 1 bis 2,5 Gew.-% Silymarin.

7. Antioxidative Zusammensetzung nach einem der Ansprüche 1-6, ferner umfassend Vitamin C, Vitamin E, Vitamin A oder Gemische davon.

8. Antioxidative Zusammensetzung nach Anspruch 7 umfassend 0,5 bis 10 Gew.-% Vitamin C und 5 bis 15 Gew.-% Vitamin E.

9. Antioxidative Zusammensetzung nach Anspruch 8 umfassend 1 bis 6 Gew.-% Vitamin C und 6 bis 14 Gew.-% Vitamin E.

10. Antioxidative Zusammensetzung nach einem der Ansprüche 1-9 zur Verwendung bei der Behandlung von oxidativer Beanspruchung in Hunden.

11. Antioxidative Zusammensetzung nach Anspruch 10 zur oralen Verabreichung, vorzugsweise in Tablettenform.

12. Dosiseinheit der antioxidativen Zusammensetzung nach einem der Ansprüche 1-7 umfassend 300-1200 mg Polyphenol-Extrakt aus Rohrzucker, 7,5-75 mg Silymarin und optional 7,5-150 mg Vitamin C und 75-225 mg Vitamin E.

13. Dosiseinheit nach Anspruch 12, umfassend 525-825 mg Polyphenol-Extrakt aus Rohrzucker und 15-37,5 mg Silymarin.

14. Dosiseinheit nach Anspruch 12 oder 13, zusätzlich umfassend 7,5-150 mg Vitamin C und 75-225 mg Vitamin E.

15. Dosiseinheit nach Anspruch 14, zusätzlich umfassend 15-90 mg Vitamin C und 90-210 mg Vitamin E.

## Revendications

1. Composition antioxydante comprenant un extrait polyphénolique de canne à sucre et de la silymarine, dans ledit extrait au moins 50 % des polyphénols ayant un poids moléculaire moyen en nombre de 600 à 1000 Daltons.

2. Composition antioxydante selon la revendication 1, dans ledit extrait au moins 70 % des polyphénols ayant un poids moléculaire moyen en nombre de 600 à 1000 Daltons.

3. Composition antioxydante selon la revendication 2, dans ledit extrait au moins 70 % des polyphénols ayant un poids moléculaire moyen en nombre de 650 à 800 Daltons.

4. Composition antioxydante selon l'une quelconque des revendications 1 à 3 comprenant 20 à 60 % en poids d'extrait polyphénolique de canne à sucre et 0,5 à 5 % en poids de silymarine.

5. Composition antioxydante selon la revendication 4 comprenant 25 à 55 % en poids d'extrait polyphénolique de canne à sucre et 1 à 3 % en poids de silymarine.

6. Composition antioxydante selon la revendication 5 comprenant 35 à 55 % en poids d'extrait polyphénolique de canne à sucre et 1 à 2,5 % en poids de silymarine.

7. Composition antioxydante selon l'une quelconque des revendications 1 à 6, comprenant en outre de la vitamine C, de la vitamine E, de la vitamine A ou des mélanges de celles-ci.

8. Composition antioxydante selon la revendication 7 comprenant 0,5 à 10 % en poids de vitamine C et 5 à 15 % en poids de vitamine E.

9. Composition antioxydante selon la revendication 8 comprenant 1 à 6 % en poids de vitamine C et 6 à 14 % en poids de vitamine E.

10. Composition antioxydante selon l'une quelconque des revendications 1 à 9 pour utilisation dans le traitement du stress oxydatif chez les chiens.

11. Composition antioxydante selon la revendication 10, destinée à être administrée par voie orale, de préférence sous forme de comprimé.

12. Dose unitaire de la composition antioxydante selon l'une quelconque des revendications 1 à 7 comprenant 300 à 1200 mg d'extrait polyphénolique de canne à sucre, 7,5 à 75 mg de silymarine, et éventuellement 7,5 à 150 mg de vitamine C et 75 à 225 mg de vitamine E.

13. Dose unitaire selon la revendication 12, comprenant 525 à 825 mg d'extrait polyphénolique de canne à sucre et 15 à 37,5 mg de silymarine.

14. Dose unitaire selon la revendication 12 ou 13, comprenant en outre 7,5 à 150 mg de vitamine C et 75 à 225 mg de vitamine E.

15. Dose unitaire selon la revendication 14, comprenant en outre 15 à 90 mg de vitamine C et 90 à 210 mg de vitamine E.
